# NOUVEAU FASCICULE DE BREVET EUROPEEN

(11) **EP 1 233 011 B2**
(45) Date de publication et mention de la décision concernant l'opposition: **06.06.2012**
(45) Mention de la délivrance du brevet: 23.05.2007
(21) Numéro de dépôt: 02290205.0
(22) Date de dépôt: 29.01.2002
(51) Int. Cl.: C07C 1/04

(54) **Procédé de synthèse d'hydrocarbures en présence d'un catalyseur comprenant un metal du groupe VIII supporte sur silice-alumine**
Verfahren zur Synthese von Kohlenwasserstoffen in Gegenwart eines Katalysators der ein Metall von Gruppe VIII auf Kieselsäure-Tonerde enthält
Process for the synthesis of hydrocarbons in presence of a catalyst comprising a metal of group VIII supported by silica-alumina

(30) Priorité: 16.02.2001 FR 0102241
(43) Date de publication de la demande: 21.08.2002
(73) Titulaire: IFP Energies nouvelles, 92852 Rueil-Malmaison Cedex (FR); ENI S.p.A., 00186 Roma (IT)
(72) Inventeur: Roy-Auberger, Magalie, 38300 Bourgoin Jallieu (FR); Courty, Philippe, 94800 Villejuif (FR); Revel, Renaud, 78800 Houilles (FR); Zennaro, Roberto, Venezia (IT)
(74) Mandataire: Boulinguiez, Didier

(56) Documents cités:
- EP-A1- 0 979 807
- EP-A1- 1 126 008
- WO-A-99/42214
- US-A- 4 497 903

## Description

La présente invention concerne un procédé de synthèse d'un mélange d'hydrocarbures linéaires et saturés contenant au moins 50% poids d'hydrocarbures C5+ et moins de 20% de méthane par rapport à l'ensemble des hydrocarbures formés, à partir d'un mélange comprenant CO-(CO₂)-H₂ (c'est à dire un mélange comprenant du monoxyde de carbone, de l'hydrogène et éventuellement du dioxyde de carbone, appelé gaz de synthèse). Ce procédé comprend la mise en oeuvre d'un catalyseur comprenant au moins un métal du groupe VIII, de préférence le cobalt, supporté sur une silice-alumine, préparée par co-précipitation et calcinée à une température comprise entre environ 500°C et environ 1200°C pendant au moins 6 heures, de manière à présenter une surface spécifique inférieure à 260 m²/g, et est caractérisé en ce que le support silice alumine est préparé selon la revendication 1.

### ART ANTERIEUR

II est connu de l'homme du métier que le gaz de synthèse peut être converti en hydrocarbures en présence de catalyseur contenant des métaux de transition. Cette conversion opérée à haute température et sous pression est connue dans la littérature sous le nom de synthèse Fischer-Tropsch. Ainsi des métaux du groupe VIII de la classification périodique des éléments tels que le fer, le ruthénium, le cobalt et le nickel catalysent la transformation de mélanges CO-(CO₂)-H₂ (c'est-à-dire un mélange de monoxyde de carbone, d'hydrogène et éventuellement de dioxyde de carbone, appelé gaz de synthèse) en hydrocarbures liquides et/ou gazeux.

Différentes méthodes ont été décrites et développées dans l'art antérieur, qui sont destinées à améliorer la préparation de catalyseurs Fischer-Tropsch à base de cobalt supporté sur différents supports. Les plus utilisés étant l'alumine, la silice et le dioxyde de titane, parfois modifiés par des éléments additionnels.

Le brevet WO 99/39825 décrit l'utilisation d'un support comprenant une base de dioxyde de titane à laquelle est incorporée un liant constitué de silice et d'alumine. Les propriétés mécaniques du catalyseur ainsi obtenu sont améliorées en particulier pour une utilisation en réacteur triphasique, généralement appelé réacteur slurry. Le plus souvent ce réacteur est du type colonne à bulle, également appelé slurry bubble column selon la terminologie anglosaxonne.

Le brevet WO 99/42214 décrit l'addition d'un élément stabilisant à un support de catalyseur partiellement soluble en milieu aqueux acide ou neutre choisi parmi l'alumine, l'oxyde de titane, la magnésie, utilisé pour la préparation d'un catalyseur actif en synthèse Fischer-Trospch. Le support préféré est l'alumine .

Le stabilisant peut être choisi dans le groupe constitué par Si, Zr, Cu, Zn, Mn, Ba, Co, Ni et/ou La. Il permet de diminuer sensiblement la solubilité du support dans des solutions aqueuses acides ou neutres. Le stabilisant préféré est la silice, ajoutée sous forme de composé organique du silicium .par dépôt sur le support d'alumine préformé ; on ajoute au moins 0,06, et au plus 2,8 atome de silicium par nanomètre carré de support.

Les catalyseurs Fischer-Trospch à base de cobalt décrits dans l'invention précitée et mis en oeuvre dans un réacteur triphasique peuvent conduire à des pertes excessives de catalyseur dans les cires paraffiniques produites, ceci par formation de fines submicroniques. Les pertes en catalyseur exprimées en cobalt peuvent atteindre 50 mg de cobalt par kilogrammes de cires.

Les brevets US 5169821 et US 5 397 806 décrivent l'inclusion de silicium, zirconium ou tantale dans un catalyseur à base de cobalt supporté sur TiO2 sous la forme d'anatase pour le stabiliser vis à vis de la régenération à haute température.

Le brevet WO 96/19289 décrit l'utilisation d'un catalyseur pour la conversion du gaz de synthèse en hydrocarbure à base de cobalt, ruthénium ou fer supporté sur un aluminosilicate cristallin mésoporeux ayant une structure poreuse bien particulière.

Le brevet US 4 497 903 décrit l'incorporation de cobalt dans les couches cristallines d'un aluminosilicate. Le catalyseur ainsi obtenu est actif pour la conversion du gaz de synthèse en hydrocarbures liquides principalement constitués d'hydrocarbures branchés à fort indice d'octane.

Les brevets ci-avant concernent la stabilisation de supports utilisés pour la production de catalyseurs de conversion du gaz de synthèse en hydrocarbures . Des brevets antérieurs doivent également être cités.

Le brevet US 4.013.590 décrit la stabilisation de supports oxydes, notamment d'alumine par addition de composés organiques du silicium séchage et décomposition thermique . Les quantités de silicium ajoutées rapportées à l'alumine correspondent à 1 à 6 atomes de silicium par nanomètre carré, les supports ainsi obtenus ont des propriétés mécaniques améliorées et sont utilisés en postcombustion automobile .

La demande de brevet européen 0.184.506 décrit la fabrication de supports de catalyseurs constitués d'agglomérés d'alumine stabilisés par addition de silice en solution aqueuse, séchage et décomposition thermique, présentant des propriétés mécaniques améliorées, notamment en termes de formation de particules fines par attrition . Ces supports sont utilisables en hydrotraitement, hydrocraquage, hydrogénation, déshydrogénation d'hydrocarbures ou d'autres composés organiques.

Le brevet US 5 045 519 décrit un procédé de préparation de silice-alumine conduisant à un produit de haute pureté, stable thermiquement. La préparation est réalisée par hydrolyse d'un alcoxyde d'aluminium et addition simultanée ou successive d'acide orthosilicique préalablement purifié par échange ionique. Les silice-alumines obtenues sont utilisables comme support de catalyseur en désulfuration, en catalyse DeNOx, en oxydation, en hydrocraquage, en hydrocraquage doux, en catalyse de post-traitement automobile, en isomérisation.

La présente invention concerne un procédé de synthèse d'un mélange d'hydrocarbures linéaires et saturés contenant au moins 50% poids d'hydrocarbures C5+ et moins de 20% de méthane par rapport à l'ensemble des hydrocarbures formés, à partir d'un mélange comprenant du monoxyde de carbone et de l'hydrogène, éventuellement du dioxyde de carbone, en présence d'un catalyseur comprenant au moins un métal du groupe VIII, de préférence le cobalt, supporté sur une silice-alumine particulière décrite ci-après. Le catalyseur est de manière préférée utilisé en suspension dans une phase liquide dans un réacteur triphasique de type autoclave parfaitement agité ou colonne à bulle (slurry bubble column). Il est également adapté à une utilisation en lit fixe.

Le demandeur a découvert que l'utilisation d'un support silice-alumine préparée par coprécipitation d'au moins un composé soluble d'aluminium et d'au moins un composé de silicium, lavage, séchage et calcination à une température élevée et pendant un temps suffisant pour favoriser les interactions entre l'alumine et la silice (liaisons Al-O-Si), permet après imprégnation d'au moins un métal du groupe VIII, de préférence le cobalt, d'obtenir un catalyseur particulièrement actif dans le procédé de synthèse d'un mélange d'hydrocarbures linéaires et saturés contenant au moins 50% poids d'hydrocarbures C5+ et moins de 20% de méthane par rapport à l'ensemble des hydrocarbures formés, à partir d'un mélange comprenant du monoxyde de carbone et de l'hydrogène. Par ailleurs ledit catalyseur présente des propriétés mécaniques améliorées, notamment lorsqu'il est de préférence mis en oeuvre en suspension dans une phase liquide dans un réacteur triphasique, ainsi qu'une meilleure résistance aux phénomènes d'attrition.

Par coprécipitation la demanderesse entend un procédé par lequel au moins un composé d'aluminium soluble en milieu neutre ou acide comme décrit ci-après, au moins un composé de silicium comme décrit ci-après, sont mis en contact, simultanément ou séquentiellement, en présence d'au moins un composé précipitant et/ou coprécipitant de façon à finalement obtenir une phase mixte essentiellement constituée de alumine-silice hydratée laquelle est éventuellement homogénéisée par agitation intense, cisaillement, broyage colloidal ou encore par combinaison de ces opérations unitaires .

Comme il est décrit ci-après la demanderesse distingue les procédés de coprécipitation vraie, à partir de solutions ou par réaction d'une solution vraie et d'une suspension colloidale submicronique par exemple d'acide silicique, en présence d'au moins un agent minéral précipitant, d'avec les procédés de coprécipitation séquencée, où un premier composé soluble en milieu acide, d'aluminium, est précipité sous forme d'hydroxide, d'oxohydroxyde ou encore d'hydroxycarbonate à partir d'un sel hydrosoluble ou encore d'un alcoxyde ou alcoolate d'aluminium puis est mélangé avec un deuxième composé de silicium et, simultanément ou consécutivement, est additionné d'au moins un agent précipîtant comme ci-après . Le composé de silicium est lui même choisi dans le groupe formé par l'acide silicique, le Ludox sous forme ammoniacale, les silicates d'ammonium quaternaires décrits dans la demande de brevet européen précitée, purs ou en mélange .Les deux modes de réalisation ci-avant constituent les procédures de coprécipitation les plus connues de l'homme de métier.

Une troisième procédure consiste à préparer par précipitation au moins un composé d'aluminium, hydroxyde, oxohydroxide, hydroxycarbonate puis à le mélanger avec au moins un composé de silicium tel qu'un acide silicique en solution aqueuse ou encore sous forme d'une suspension colloidale submicronique, ou hydrosol. Le mélange obtenu est alors homogénéisé à l'échelle micronique et de préférence à l'échelle nanométrique par agitation intense, cisaillement, broyage colloidal ou combinaison de ces opérations unitaires connues de l'homme de métier. Ce mode de réalisation est assimilé à une coprécipitation séquencée.

La silice-alumine utilisée dans le procédé selon l'invention est de préférence une silice-alumine homogène à l'échelle du micromètre et dans laquelle la teneur en impuretés anioniques (par exemple SO₄²⁻, Cl⁻) et cationiques (par exemple Na⁺) est de préférence inférieure à 0,1% poids, de manière plus préférée inférieure à 0,05 % poids.

La silice-alumine utilisée dans le procédé selon l'invention est préparée par coprécipitation conformément à la revendication 1.

Après coprécipitation, le support est obtenu par filtration et lavage, éventuellement lavage par une solution ammoniacale pour extraire par échange ionique le sodium résiduel, séchage avec mise en forme par exemple par atomisation puis calcination préférentiellement sous air en four rotatif et à température élevée, comprise entre environ 500°C et environ 1200°C, pendant au moins 6 heures, temps suffisant pour favoriser la formation d'interactions entre l'alumine et la silice. Ces interactions conduisent en effet à une meilleure résistance mécanique du support et donc du catalyseur utilisé dans le procédé selon l'invention.

La méthode de préparation de silice-alumine selon l'invention consiste à précipiter l'hydrate d'alumine comme ci-avant, à le laver puis à le mélanger avec l'acide orthosilicique aqueux de façon à obtenir une suspension, laquelle est intimement homogénéisée par forte agitation et cisaillement. Une turbine Ultraturrax ou encore une turbine Staro peut être utilisée, ou encore un broyeur colloidal par exemple, un broyeur colloidal IKA . La suspension homogène est alors séchée par atomisation comme ci-avant puis calcinée entre environ 500°C et environ 1200°C pendant au moins 6 heures, un support silice-alumine utilisable dans le procédé selon l'invention est ainsi obtenu

Il peut être éventuellement souhaitable d'ajouter, lors d'une étape quelconque de la préparation, une proportion mineure d'au moins un élément stabilisant choisi dans le groupe formé par le lanthane, le praséodyme, le néodyme. L'élément stabilisant est de préférence ajouté sous forme d'un sel soluble, par exemple un nitrate.

D'une façon préférée, un sel soluble d'au moins un élément stabilisant est ajouté dans la solution aqueuse de sel d'aluminium cationique ou encore, comme enseigné dans le brevet US 5 045 519 simultanément ou consécutivement à la mise en contact du composé d'aluminium et d'acide orthosilicique en milieu aqueux, l'acide orthosilicique étant lui même ajouté simultanément ou consécutivement à l"hydrolyse d'au moins un alkoxyde d'aluminium.

Les précurseurs d'alumine utilisés selon la présente invention se distingue donc de ceux cités dans l'art antérieur par les caractéristiques suivantes :

Ils sont entièrement soluble en milieu aqueux : sels cationiques d'aluminium, par exemple le nitrate, ou encore en milieu organique, par exemple: l'hexanoate d'aluminium en milieu hexanol .

Ils sont solubles en milieu acide, et sont constitués par au moins un hydrogel d'alumine comme par exemple les hydroxydes ou encore les oxohydroxides d'aluminium comme par exemple les hydrates d'alumine tels que le trihydroxide microcristallisé ou amorphe, la pseudoboehmite, la boehmite, la bayerite, l'hydrargillite, le diaspore ; ou encore par au moins un hydroxycarbonate d'aluminium Par solubles en milieu acide la demanderesse entend que leur mise en contact avant toute addition de silicium, immédiatement après coprécipitation et lavage, avec une solution acide par exemple d'acide sulfurique ou encore d'acide nitrique provoque leur dissolution totale ou, s'ils sont essentiellement cristallisés, la formation d'un hydrosol submicronique de boehmite où l'essentiel de l'alumine est apparemment dissout . Cette propriété de dissolution est une propriété recherchée de l'invention, elle s'applique aux hydrogels d'alumine avant addition du silicium

Ils ne sont mis en forme qu'après mise en contact avec au moins un composé de silice et optionnellement d'autres métaux comme ci-après .
Ils se distinguent ainsi des alumines activées telles que les alumines gamma, delta, théta, éta, alpha pures ou en mélange, lesquelles sont au plus partiellement solubles en milieu aqueux neutre ou préférentiellement acide comme enseigné dans l'art antérieur précité et qui sont mises en forme préalablement à l'addition de silicium lorsqu'elles sont ensuite additionnées de cobalt et utilisées en synthèse d'hydrocarbures à partir de gaz de synthèse . En outre dans ce cas qui ne fait pas partie de la présente invention, la propriété recherchée est une dissolution minimale du dit support.

Les supports de silice-alumine utilisés dans la présente invention contiennent de préférence entre 0,5 et 30 %poids de silice, de manière plus préférée entre de 1 à 20 %poids, de manière encore plus préférée entre 1,4 et 15 %poids de silice par rapport au produit anhydre.

Ils peuvent en outre contenir de 0,1 à 5 %poids, de préférence de 0,5 à 2 %poids d'au moins un oxyde M2O3 d'au moins un métal M choisi dans le groupe formé par le lanthane, le praséodyme et le néodyme.
Le support est préférentiellement mis en forme sous la forme d'une poudre fine calibrée présentant une taille de grain inférieure à 800 microns (µm), de préférence entre 10 et 500 µm, de manière plus préférée entre 10 et 300 µm, et de manière très préférée entre 20 et 150 µm, pour une utilisation optimale en présence d'une phase liquide. L'étape unitaire de séchage avec mise en forme est réalisée de manière préférée par atomisation, qui permet d'obtenir des microbilles sensiblement sphériques de taille inférieure à environ 800 µm.

Après séchage, le produit est calciné par exemple sous air et dans un four rotatif à une température comprise entre environ 500°C et environ 1200°C de préférence entre 550°C et 1200°C, de manière plus préférée entre 700°C et 1200°C, et de manière très préférée entre 800°C et 1100°C, et pendant un temps suffisant pour amener sa surface spécifique à moins de 260 m²/g, préférentiellement à moins de 220 m²/g, de manière plus préférée à une surface spécifique comprise entre 130 et 200 m²/g, et de manière très préférée entre 130 et 190 m²/g. Ladite étape de calcination dure au moins 6 heures, de préférence au moins 10 heures, de manière préférée au moins 15 heures. Par exemple, ladite silice-alumine peut être calcinée pendant 12 heures à 1050°C.

Il est également possible de débuter la calcination à basse température, c'est-à-dire à une température comprise entre 350°C et 550°C pendant au moins 1 heure, de préférence au moins 3 heures, puis de l'achever à une température comprise entre environ 500°C et environ 1200°C

Dans une autre mise en oeuvre, le support est calciné dans un premier temps à 550°C pendant trois heures puis traité sous un mélange air /H2O à 800°C pendant 24 heures, afin d'obtenir la surface spécifique recherchée.

Dans une autre variante, le support se présente sous la forme de sphères ou d'extrudés de diamètre équivalent compris entre 2 et 10 mm, pour une mise en oeuvre en lit fixe.

Les supports de catalyseur utilisables dans la présente invention présentent une homogénéité micronique (c'est-à-dire à l'échelle du micron), déterminée par microanalyse à la microsonde de Castaing, telle que le rapport atomique Si/Al mesurée localement dans plusieurs coupes de particules de support ne fluctue pas de plus de 20 % par rapport à la valeur moyenne.

D'une façon préférée, les supports conduisant aux catalyseurs utilisés dans l'invention présentent une homogénéité 'nanométrique', c'est-à-dire à l'échelle du nanomètre.

Une méthode qui peut être utilisée pour caractériser les supports et en particulier déterminer leur homogénéité, est la microscopie électronique par transmission (MET). Pour cela on utilise un microscope électronique (du type Jeol 2010 ou Philips Tecnai20F éventuellement avec balayage) équipé d'un spectromètre à dispersion d'énergie (EDS) pour l'analyse des rayons X (par exemple un Tracor ou un Edax). Le détecteur EDS doit permettre la détection des éléments légers. L'association de ces deux outils, MET et EDS, permet de combiner l'imagerie et l'analyse chimique locale avec une bonne résolution spatiale.

Pour ce type d'analyse, les échantillons sont finement broyés à sec dans un mortier ; la poudre est ensuite incluse dans de la résine pour réaliser des coupes ultrafines d'épaisseur 70 nm (nanomètres) environ. Ces coupes sont recueillies sur des grilles de cuivre, recouvertes d'un film de carbone amorphe à trous, et servant de support. Elles sont ensuite introduites dans le microscope pour observation et analyse sous vide secondaire. En imagerie, on distingue alors aisément les zones d'échantillon des zones de résine. On procède ensuite à un certains nombre d'analyses, 10 au minimum, de préférence comprises entre 15 et 30, sur différentes zones de l'échantillon inclus. La taille du faisceau électronique pour l'analyse des zones (déterminant approximativement la taille des zones analysées) est de 50 nm de diamètre au maximum, et de préférence de 20 nm, de manière encore plus préférée 10, 5, 2 ou 1 nm de diamètre. En mode balayé, la zone analysée sera fonction de la taille de la zone balayée et non plus de la taille du faisceau généralement réduit.
Le traitement semi quantitatif des spectres X recueillis à l'aide du spectromètre EDS permet d'obtenir la concentration relative de Al et de Si (en % atomique) et le rapport Si/Al pour chacune des zones analysées. On peut alors calculer la moyenne Si/Alₘ et l'écart type σ de cet ensemble de mesures.
La présente invention fait état de catalyseurs dont l'écart type σ relatif (par rapport à la valeur Si/Alₘ), est inférieur à 30%, de préférence 20% pour un rapport global Si/Al de préférence compris entre 0,1 et 10. Ce rapport global peut être mesuré par d'autres techniques couramment utilisées pour ce type d'analyse (fluorescence X par exemple).

Le catalyseur utilisé dans le procédé selon l'invention est constitué d'au moins un élément du groupe VIII (élément choisi parmi les éléments des groupes 8, 9 et 10 de la nouvelle classification périodique), supporté sur une silice-alumine présentant les caractéristiques décrites ci-avant, et préparée par coprécipitation.

L'élément du groupe VIII de la classification périodique est de préférence choisi parmi le fer, le cobalt et le ruthénium. De manière plus préférée, le métal du groupe VIII est le cobalt.

Une technique de préparation du catalyseur qui convient particulièrement pour la mise en oeuvre de l'invention est l'imprégnation d'une solution aqueuse d'un précurseur du métal du groupe VIII de la classification périodique des éléments, de préférence le cobalt, par exemple une solution aqueuse de sels tels que les nitrates de cobalt. La teneur en poids par rapport au poids total de catalyseur en métal du groupe VIII est généralement comprise entre 0,1 et 50%, préférentiellement entre 1 et 30%.

Le catalyseur peut aussi contenir au moins un élément additionnel, par exemple un promoteur d'activité, tel que par exemple au moins un élément choisi dans le groupe constitué par le ruthénium, le molybdène et le tantale ou des promoteurs de réductibilité tels que par exemple le platine, le palladium ou le ruthénium. La teneur en poids d'un élément additionnel par rapport au poids total de catalyseur est généralement comprise entre 0,01 et 5%. Ces éléments additionnels sont de préférence introduits en même temps que le métal du groupe VIII ou, selon une autre variante préférée, dans au moins une étape ultérieure.

Dans un mode particulier de réalisation de l'invention, le catalyseur contient à la fois du cobalt et du ruthénium. Dans un autre mode particulier de réalisation de l'invention le catalyseur contient du cobalt et du tantale.

La catalyseur selon l'invention, présente une résistance mécanique améliorée par rapport à un catalyseur comprenant un support constitué uniquement d'alumine ou de silice, ou de dioxyde de titane.
La mesure de la résistance mécanique du catalyseur selon l'invention peut être réalisée par mesure de l'évolution de la taille des particules à l'issue d'une durée de test déterminée lors d'une mise en oeuvre en réacteur triphasique.

Les catalyseurs ainsi préparés conduisent à des performances particulièrement stables en synthèse Fischer-Tropsch et à la conversion du gaz de synthèse en un mélange d'hydrocarbures linéaires et saturés contenant au moins 50% poids d'hydrocarbures C5+ et moins de 20% de méthane par rapport à l'ensemble des hydrocarbures formés.

Les conditions de mise en oeuvre desdits catalyseurs pour la synthèse d'hydrocarbures sont habituellement les suivantes :
Le catalyseur comprenant au moins un métal du groupe VIII imprégné sur le support silice alumine précédemment décrit est séché puis calciné. Le catalyseur est ensuite pré-réduit par au moins un composé réducteur, par exemple choisi dans le groupe constitué par l'hydrogène, le monoxyde de carbone et l'acide formique, éventuellement mélangé avec un gaz inerte, l'azote par exemple, dans un rapport molaire composé réducteur/(composé réducteur + gaz inerte) compris entre 0,001 : 1 à 1:1.

La réduction peut être menée en phase gazeuse à une température comprise entre 100°C et 600°C, de préférence entre 150°C et 400°C, et une pression comprise entre 0,1 et 10 MPa et à une vitesse volumétrique horaire comprise entre 100 et 40 000 volumes de mélange par volume de catalyseur et par heure.

Cette réduction peut également être menée en phase liquide, dans les mêmes conditions opératoires qu'en phase gazeuse, le catalyseur est alors mis en suspension dans une phase liquide inerte (également appelée solvant), par exemple une coupe paraffinique comprenant au moins un hydrocarbure ayant au moins 5, de préférence au moins 10 atomes de carbone par molécule.

Lorsque le catalyseur est mis en oeuvre dans un réacteur triphasique, il sera avantageux d'utiliser de préférence le même solvant inerte que celui utilisé pendant la réaction. De manière très préférée, on utilisera donc une coupe paraffinique issue de la synthèse Fischer-Tropsch, par exemple une coupe kérosène ou gasoil. De manière préférée, cette réduction sera menée in-situ, c'est-à-dire dans le réacteur qui est ensuite utilisé pour réaliser la synthèse Fischer-Tropsch.

La réduction du catalyseur utilisé dans le procédé selon l'invention peut également être menée ex-situ ou « hors site », c'est à dire en dehors du réacteur de synthèse Fischer-Tropsch, voire en dehors du site industriel de mise en oeuvre du procédé. Le réduction pourra ainsi être éventuellement mise en oeuvre dans une sociétés habilitée à opérer des traitements « hors site » de catalyseurs.

Dans un tel cas, le catalyseur sera réduit dans les conditions opératoires décrites ci-avant. Après réduction et refroidissement à moins de 100°C du catalyseur réduit, ledit catalyseur est préférentiellement mélangé, à raison de 10% à 80% poids, avec des cires paraffiniques solides à température ambiante et préchauffées afin de rendre ces cires liquides. De manière préférée, on utilise des cires paraffiniques issues d'une synthèse Fischer-Tropsch. Après mélange, la suspension obtenue est coagulée en gouttes par projection sur un tapis porteur, suivie d'un refroidissement.
Le produit obtenu est sous forme de grains présentant un diamètre équivalent (diamêtre de la sphère de volume équivalent) compris entre environ 5 et environ 20 mm de diamêtre. Ces grains de catalyseur peuvent être chargés directement dans le réacteur Fischer-Tropsch.

La conversion du gaz de synthèse en hydrocarbures est ensuite opérée sous une pression totale habituellement comprise entre 0,1 et 15 MPa et de préférence entre 1 et 10 MPa, la température est généralement comprise entre 150 et 350°C et de préférence entre 170 et 300°C. La vitesse volumétrique horaire est habituellement comprise entre 100 et 20000 volumes de gaz de synthèse par volume de catalyseur et par heure (h⁻¹) et de préférence entre 200 et 10000 h⁻¹, de manière plus préférée entre 400 et 5000 h⁻¹, et le rapport H2/CO dans le gaz de synthèse est habituellement compris entre 1:2 et 5:1, de préférence entre 1,2:1 et 2,5:1.

Le catalyseur peut-être utilisé sous la forme d'une poudre fine calibrée présentant une taille de grain inférieure à 800 microns (µm), de préférence entre 10 et 500 µm, de manière plus préférée entre 10 et 300 µm, et de manière très préférée entre 20 et 150 µm, de manière encore plus préférée entre 20 et 120 µm, lorsqu'il est mis en oeuvre en suspension dans une phase liquide. Il peut également être utilisé sous la forme de particules de diamètre équivalent compris entre 2 et 10 mm environ, de préférence compris entre 3 et 8 mm, lorsqu'il est mis en oeuvre en lit fixe.

Le procédé selon l'invention peut être mis en oeuvre avec ledit catalyseur disposé en lit fixe. Dans un tel procédé, la réaction a lieu en phase gazeuse. Le catalyseur décrit précédemment présente une résistance mécanique suffisamment élevée peut être manipulé et chargé dans un tel réacteur sans risque de désagrégation. Le procédé selon l'invention peut également, de préférence être mis en oeuvre au moyen d'un réacteur triphasique dans lequel le catalyseur est mis en suspension dans une phase liquide inerte (solvant). Par exemple, un réacteur parfaitement agité tel qu'un autoclave ou un réacteur triphasique de type colonne à bulle (également appelé slurry bubble column).

En effet, le catalyseur est avantageusement employé dans un réacteur triphasique, de préférence de type colonne à bulles, car ce type de mise en oeuvre permet :
- une utilisation optimale des performances du catalyseur (activité et sélectivité), par limitation des phénomènes diffusionnels intra-granulaires.
- une limitation très importante des effets thermiques dans le grain de catalyseur, qui est entouré d'une phase liquide
Ce type de mise en oeuvre impose une séparation du catalyseur et des produits de réaction.
Dans ces conditions, le catalyseur utilisé dans le procédé selon l'invention présente des propriétés mécaniques améliorées, ce qui conduit à une séparation du catalyseur et des produits optimale, et une durée de vie augmentée. Ledit catalyseur présente en particulier une résistance à l'attrition améliorée, d'où une diminution très significative de la quantité de fines formée lors d'une mise en oeuvre en réacteur triphasique. Une explication possible de cette amélioration est la présence d'interactions plus importantes et plus nombreuses entre l'alumine et la silice dans le support silice-alumine préparé par coprécipitation.

En résumé, l'invention concerne un procédé de synthèse d'un mélange d'hydrocarbures linéaires et saturés contenant au moins 50% poids d'hydrocarbures C5+ et moins de 20% de méthane par rapport à l'ensemble des hydrocarbures formés, à partir d'un mélange comprenant du monoxyde de carbone et de l'hydrogène, en présence d'un catalyseur comprenant au moins un métal du groupe VIII supporté sur une silice-alumine préparée par coprécipitation et calcinée à une température comprise entre environ 500°C et environ 1200°C pendant au moins 6 heures, de manière à ce que ladite silice alumine présente une surface spécifique inférieure à 260 m2/g et est caractérisé en ce que le support silice alumine est préparé selon la revendication 1.

Selon un mode préféré, la silice-alumine est calcinée à une température comprise entre 700°C et 1200°C pendant au moins 10 heures. Selon un autre mode préféré, la silice-alumine est tout d'abord calcinée à une température comprise entre environ 350°C et environ 550°C pendant au moins 1 heure, puis à une température comprise entre environ 500°C et environ 1200°C pendant au moins 6 heures

De préférence, la silice-alumine est homogène à l'échelle du micromètre, et de manière plus préférée, la teneur en impuretés anioniques et cationiques est inférieure à 0,1 % poids.

De préférence, la silice-alumine contient entre 0,5 % et 30 % poids de silice par rapport au produit anhydre, et la teneur en métal du groupe VIII est comprise entre 0,1 et 50 % poids. Le métal du groupe VIII est de préférence le cobalt.

Le catalyseur du procédé selon l'invention peut éventuellement contenir en outre au moins un élément additionnel choisi dans le groupe constitué par: le ruthénium, le molybdène, le tantale, le platine, le palladium. Il peut également contenir en outre de 0,1 à 5 %poids d'au moins un oxyde M2O3 d'au moins un métal M choisi dans le groupe constitué par le lanthane, le praséodyme et le néodyme.
De manière préférée, le catalyseur est mis en oeuvre en suspension dans une phase liquide, dans un réacteur triphasique, généralement sous la forme d'une poudre présentant une taille de grain inférieure à 800 microns. Ledit catalyseur peut toutefois être mis en oeuvre en lit fixe sous la forme de particules de diamètre équivalent compris entre 2 et 10 mm.

Les exemples qui suivent illustrent l'invention.

### Exemple 1 (comparatif) : catalyseur A

Un catalyseur A, Co/SiO2-Al2O3 est préparé par imprégnation de nitrate de cobalt sur une poudre silice-alumine. La silice-alumine est préalablement préparée par co-précipitation de silicate de sodium, aluminate de sodium, sulfate d'aluminium et acide sulfurique de manière à obtenir une composition finale égale à SiO₂/Al₂O₃ = 5/95, et une surface spécifique de 220 m²/g après calcination pendant 6 heures à 600 °C. La suspension obtenue est atomisée et le support ainsi obtenu se présente sous forme de microbilles de taille comprise entre 20 et 150 microns.
Le catalyseur issu de l'imprégnation est séché et calciné à 400°C pendant 2 heures. La teneur en cobalt métalllique est de 13 % poids.

### Exemple 2 (comparatif) : catalyseur B

Un catalyseur B, Co/SiO2-Al2O3 est préparé par imprégnation de nitrate de cobalt sur une silice-alumine préparée par coprécipitation d'un mélange d'acide silicique H2SiO4 et de nitrate d'aluminium, auquel est ajouté de l'ammoniaque. Après atomisation, le support obtenu se présente sous forme de microbilles de 40 à 120 microns.
La composition de la silice-alumine après calcination à 700°C pendant 6 heures est égale à SiO₂/Al₂O₃ = 10/95 et sa surface spécifique est égale à 170 m²/g.
Le catalyseur issu de l'imprégnation est séché et calciné à 400°C pendant 2 heures. La teneur en cobalt métallique est égale à 12,5% poids

### Exemple 3 (comparatif) : Catalyseur C

Un catalyseur C, Co/SiO2-Al2O3 est préparé par imprégnation de nitrate de cobalt sur une silice-alumine de type siralox 5 (commercialisé par la société Condea) préparé par addition d'acide silicique à un alcoxyde d'aluminium hydrolysé, puis atomisé. Le support ainsi obtenu est calciné à 1000°C pendant 12 h, sa surface spécifique est de 150 m2/g et la teneur en poids de SiO2 égale à 5 %. Le spectre infrarouge des hydroxyles obtenus après pastillage du support seul et traitement sous vide à 500°C montre la présence d'une bande à 3750 cm⁻¹ relative aux SiOH à laquelle s'ajoute un large massif entre 3750 et 3725 cm⁻¹ relative aux AI-OH, d'intensité plus faible.
Le catalyseur issu de l'imprégnation est séché et calciné à 400°C pendant 2 heures. La teneur en cobalt métallique est égale à 12,5% poids.

### Exemple 4 (comparatif) : catalyseur D

Un catalyseur D est préparé de la même façon que le catalyseur C, du nitrate de Lanthane est ajouté en même temps que l'acide silicique de manière à obtenir un catalyseur contenant 12 % poids de cobalt sur un support constitué par 2 % poids d'oxyde de lanthane, 5 % poids de silice et 93 % poids d'alumine. Sa surface spécifique est de 145 m²/g après calcination pendant 2 heures à 400 °C.

### Exemple 5 (comparatif) : catalyseur E

Un catalyseur E, Co/Al2O3 est préparé par imprégnation de nitrate de cobalt sur une poudre d'alumine Puralox Scca 5-170 de surface spécifique égale à 180 m²/g. Ce support se présente sous forme de microbilles de granulométrie comprise entre 20 et 150 microns.
Le catalyseur issu de l'imprégnation est séché et calciné à 400°C pendant 2 heures. La teneur finale en cobalt est de 12,5 % poids.

### Exemple 6 (comparatif) : catalyseur F

Un catalyseur F est préparé au moyen des étapes successives suivantes :
1- imprégnation de TEOS (tétraéthoxysilane) sur des microbilles d'alumine puralox scca 5-170 de surface spécifique égale à 180 m2/g ; selon la méthode décrite par B. BEGUIN, E. GARBOWSKI et M. PRIMET dans « Journal of Catalysis », page 595, volume 127, 1991,
2- calcination à 500°C pendant 2 heures.
3- imprégnation de nitrate de cobalt, séchage et calcination à 400°C pendant 2 heures.
Par ailleurs, le spectre infrarouge des hydroxyles obtenu après pastillage du support modifié seul et traitement sous vide à 500°C montre seulement la présence d'une bande hydroxyle à 3745 cm⁻¹ relative aux Si-OH, les bandes des hydroxyles de l'alumine à 3760, 3730, 3685 et 3580 cm⁻¹, ont disparu.

Le catalyseur F contient 13 % poids de cobalt et 3 % poids de silice sur un support constitué d'alumine.

### Exemple 7 (comparatif) : catalyseur G

Un catalyseur G, Co/SiO2-Al203 est préparé par imprégnation de nitrate de cobalt sur une poudre silice-alumine.

La silice-alumine est préparée par addition d'acide silicique à un gel d'alumine lui-même obtenu par hydrolyse d'un alcoxyde d'aluminium.
Le support obtenu est calciné à 500°C pendant 4 heures, sa surface spécifique est de 410 m²/g et il comprend 20 % poids de silice et 80 % poids d'alumine.
Le catalyseur obtenu après imprégnation du nitrate de cobalt est séché puis calciné à 400°C pendant 2 heures.
L'a teneur en cobalt du catalyseur est de 13 % poids.

### Exemple 7 bis (selon l'invention) : catalyseur H

Un catalyseur H, Co/SiO2-Al2O3 est préparé par imprégnation de nitrate de cobalt sur une poudre calibrée de silice alumine.
La poudre de silice alumine est obtenue par précipitation d'hydrargillite en milieu alcalin, lavage en présence d'ammoniaque jusqu'à désalcalinisation poussée puis mélange intime par passage dans un broyeur colloidal IKA en présence de CO2 avec une solution d'acide orthosilicique obtenue elle même par décationisation d'une solution de silicate alcalin, jusqu'à obtention d'une suspension homogène laquelle est alors séchée par atomisation puis calcinée à 1100°C 6 heures . On obtient une silice-alumine de surface spécifique 170 m2/g
Le support est alors imprégné de nitrate de cobalt, séché à 120°C pendant 6 heures, calciné à 400°C sous azote pendant 3 heures.

Le catalyseur H contient 12,8%poids de cobalt et 7% poids de silice sur un support constitué de silice-alumine .

### Exemple 7 ter : Tests comparatifs de dissolution

10,2 grammes d'alumine Al2O3 sous forme d'hydrate Al(OH)3 de structure hydrargillite préparée selon l'exemple 7 bis, sont mis en contact 2 heures à 80° puis 110°C avec 60 millilitres d'une solution aqueuse d'acide sulfurique contenant 80 grammes d'acide pur . A l'issue des 2 heures l'hydrate d'alumine est totalement dissous et forme une solution de sulfate d'aluminium limpide .
Séparément 10,2 grammes d'alumine Al2O3 sous forme de Puralox SCCA-5-170 utilisée dans l'exemple comparatif 6 sont mis en contact 2 heures à 80 puis 110°C avec 60 millilitres d'une solution aqueuse d'acide sulfurique contenant 80 grammes d'acide pur . A l'issue des 2 heures le liquide surnageant est filtré . La quantité totale d'aluminium dans le filtrat exprimée en alumine est inférieure à 0.5 gramme .
Il apparaît ainsi que dans des conditions opératoires où l'alumine activée utilisée dans l'exemple comparatif 6 est partiellement soluble, les hydrates ou gels d'alumine tels que cités dans la présente invention sont totalement solubles .

### Exemple 8 : Tests catalytiques en réacteur à lit fixe

Les catalyseurs A à F dont les préparations sont décrites dans les exemples 1 à 6 sont testés en lit fixe phase gazeuse dans une unité fonctionnant en continu et opérant sur 20 cm³ de catalyseur. Les catalyseurs sont préalablement réduits in situ à 350°C pendant 12 heures sous un mélange d'hydrogène et d'azote contenant 30% d'hydrogène, puis pendant 12 heures sous hydrogène pur.
Les conditions de test des catalyseurs sont les suivantes :
- T°C = 220°C,
- Pression = 2MPa
- vitesse volumique horaire (VVH)=1500 h⁻¹
- rapport molaire H₂/CO = 2/1,

**TABLEAU 1 : Conversion du gaz de synthèse en hydrocarbures**

| **Catalyseur** | **Conv CO (%vol après 100h)** | **Distribution des produits formés (% poids)** | |
|---|---|---|---|
| | | **C1** | **C5+** |
| A (comparatif) | 65 | **12** | **77** |
| B (comparatif) | 65 | **13** | **75** |
| C (comparatif) | 62 | **10** | **76** |
| D (comparatif) | 62 | **9** | **79** |
| E(comparatif) | 68 | **16** | **54** |
| F(comparatif) | 68 | **21** | **64,5** |

### Exemple 9 : Tests catalytiques en réacteur triphasique

Les catalyseurs A à G décrits dans les exemples 1 à 8 ci-dessus sont testés dans un réacteur triphasique, parfaitement agité, fonctionnant en continu et opérant avec une concentration de 10% (mol) de catalyseur en suspension.
Les conditions de test sont les suivantes :
- T°C = 230°C,
- Pression = 2 MPa
- vitesse volumique horaire (VVH) = 1000 h⁻¹
- rapport molaire H₂/CO = 2/1

**TABLEAU 2 : Conversion du gaz de synthèse en hydrocarbures**

| **Catalyseur** | **Conv CO (%vol après 100h)** | **Distribution des produits formés (% poids)** | |
|---|---|---|---|
| | | **C1** | **C5+** |
| A (comparatif) | 55 | **9** | **78** |
| B (comparatif) | 55 | **10** | **77** |
| C (comparatif) | 53 | **9** | **77** |
| D (comparatif) | 53 | **8** | **77** |
| E (comparatif) | 50 | **11** | **64** |
| F (comparatif) | 49 | **12** | **66** |
| G (comparatif) | 50 | **15** | **63** |

Après 500 heures de test, la résistance mécanique des catalyseurs a été évaluée par mesure de la granulométrie des catalyseurs obtenus après séparation des produits de réaction. Le tableau 3 ci-dessous donne le % de particules de catalyseur présentant une taille inférieures à 20 microns formées lors des test des catalyseurs.

**TABLEAU 3 : Résistance à l'attrition**

| **Catalyseur** | **% particules inférieures à 20 microns** |
|---|---|
| | |
| A (comparatif) | 5 |
| B (comparatif) | 3 |
| C(comparatif) | 4 |
| D(comparatif) | 4 |
| E (comparatif) | 10 |
| F(comparatif) | 9 |
| G (comparatif) | 9 |
| H(invention) | 3,5 |

Le catalyseur utilisé dans le procédé selon l'invention (H) présente une résistance mécanique nettement supérieure par rapport aux catalyseurs E, F et G.

### Exemple 10 :

Les catalyseurs A et E décrits dans les exemples 1 et 5 ci-avant sont mis en oeuvre dans un appareillage comprenant (schéma figure 1) :
- une colonne à bulles de diamètre interne 50 mm et hauteur 1500 mm,
- une tubulure d'admission du gaz de synthèse, en bas de colonne,
- une tubulure de soutirage de la suspension , au dessus du niveau liquide,
- une tubulure de réinjection de la suspension, en bas de colonne,
- une boucle de circulation, comprenant un dégazeur, un décanteur, et une pompe.

Le catalyseur A, introduit dans la colonne à raison de 500 g dans 1,5 de paraffine n
- C18 est utilisé dans les conditions suivantes :
   débit de charge : 1 m³/h mélange CO : H2 (rapport molaire 1 :2)
      T = 220°C
      P= 2MPa.

La conversion du CO est de 72%, la sélectivité en C5+ de 77% poids. Le catalyseur est séparé des produits au moyen du décanteur, la teneur en catalyseur dans le liquide est de moins de 250 ppm poids, soit environ 33 ppm de cobalt, ce qui ne nécessite qu'une filtration très simple, sans obligation de recycler les très faibles quantités de catalyseur filtrées vers le réacteur.
Le catalyseur E, mis en oeuvre dans les mêmes conditions que A, conduit à une conversion de CO de 65 %, et une sélectivité en C5+ de 76 %. La décantation dans le même appareil laisse 0,15 % poids de catalyseur dans le liquide, soit environ 180 ppm de cobalt, ce qui nécessite une filtration ultérieure plus complexe et un recyclage du catalyseur séparé au réacteur.

## Revendications

1. Procédé de synthèse d'un mélange d'hydrocarbures linéaires et saturés contenant au moins 50% poids d'hydrocarbures C5+ et moins de 20% de méthane par rapport à l'ensemble des hydrocarbures formés, à partir d'un mélange comprenant du monoxyde de carbone et de l'hydrogène en présence d'un catalyseur comprenant au moins un métal du groupe VIII supporté sur une silice-alumine préparée par coprécipitation et calcinée à une température comprise entre environ 500°C et environ 1200°C pendant au moins 6 heures, de manière à ce que ladite silice alumine présente une surface spécifique inférieure à 260 m2/g, **caractérisé en ce que** le support silice alumine est préparé par précipitation d'un hydrogel d'alumine puis où l'hydrogel mixte silice alumine est obtenu par addition d'une solution d'acide silicique, homogénéisation poussée par forte agitation, cisaillement, broyage colloïdal, séchage avec mise en forme puis calcination.

2. Procédé selon la revendication 1, dans lequel l'homogénéisation est menée à bien par ultra broyage dans un broyeur colloïdal.

3. Procédé selon l'une quelconque des revendications 1 ou 2 dans lequel la silice-alumine est calcinée à une température comprise entre 700°C et 1200°C pendant au moins 10 heures.

4. Procédé selon selon l'une quelconque des revendications 1 à 3 dans lequel la silice-alumine est tout d'abord calcinée à une température comprise entre 350°C et 550°C pendant au moins 1 heure, puis à une température comprise entre environ 500°C et environ 1200°C pendant au moins 6 heures.

5. Procédé selon l'une quelconque des revendications 1 à 4 dans lequel la silice-alumine est homogène à l'échelle du micromètre.

6. Procédé selon l'une quelconque des revendications 1 à 5 dans lequel la silice-alumine est homogène à l'échelle du nanomètre.

7. Procédé selon l'une quelconque des revendications 1 à 6 dans lequel la teneur en impuretés anioniques et cationiques est inférieure à 0,1 % poids.

8. Procédé selon l'une quelconque des revendications 1 à 7 dans lequel la silice-alumine contient entre 0,5 % et 30 % poids de silice par rapport au produit anhydre.

9. Procédé selon l'une quelconque des revendications 1 à 8 dans lequel la teneur en métal du groupe VIII est comprise entre 0,1 et 50 % poids.

10. Procédé selon l'une quelconque des revendications 1 à 9 dans lequel le métal du groupe VIII est le cobalt.

11. Procédé selon l'une quelconque des revendications 1 à 10 dans lequel le catalyseur contient au moins un élément additionnel choisi dans le groupe constitué par le ruthénium, le molybdène, le tantale, le platine, le palladium.

12. Procédé selon l'une quelconque des revendications 1 à 11 dans lequel le catalyseur contient en outre 0,1 à 5% poids d'au moins un oxyde M203 d'au moins un métal M choisi dans le groupe constitué par le lanthane, le praséodyme et le néodyme.

13. Procédé selon l'une quelconque des revendications 1 à 12 dans lequel le catalyseur est mis en oeuvre en suspension dans une phase liquide, dans un réacteur triphasique.

14. Procédé selon la revendication 13 dans lequel ledit catalyseur est sous la forme d'une poudre présentant une taille de grain inférieure à 800 microns.

15. Procédé selon l'une quelconque des revendications 1 à 12 dans lequel le catalyseur est mis en oeuvre en lit fixe, sous la forme de particules de diamètre équivalent compris entre 2 et 10 mm.

## Claims

1. A process for synthesising for synthesising a mixture of linear and saturated hydrocarbons containing at least 50 wt% of C5+ hydrocarbons and less than 20% of methane with respect to the total hydrocarbons formed, from a mixture comprising carbon monoxide and hydrogen in the presence of a catalyst comprising at least one group VIII metal supported on a silica-alumina prepared by co-precipitating and calcining at a temperature in the range from about 500°C to about 1200°C for at least 6 hours such that said silica-alumina has a specific surface area of less than 260 m²/g, **characterized in that** the silica alumina support is prepared by precipitation of an alumina hydrogel then wherein the mixed silica alumina hydrogel is obtained by adding a solution of silicic acid, intense homogenisation by vigorous stirring, shearing, colloidal milling, drying with forming then calcining.

2. A process according to claim 1, wherein homogenisation is carried out by ultramilling in a colloidal mill.

3. A process according to any one of claims 1 to 2, wherein the silica-alumina is calcined at a temperature in the range 700°C to 1200°C for at least 10 hours.

4. A process according to any one of claims 1 to 3, wherein the silica-alumina is initially calcined at a temperature in the range of 350°C to 550°C for at least 1 hour, then at a temperature in the range of about 500°C to about 1200°C for at least 6 hours.

5. A process according to any one of claims 1 to 4, wherein the silica-alumina is homogeneous on the micrometre scale.

6. A process according to any one of claims 1 to 5, wherein the silica-alumina is homogeneous on the nanometre scale.

7. A process according to any one of claims 1 to 6, wherein the amount of anionic and cationic impurities is less than 0.1 % by weight.

8. A process according to any one of claims 1 to 7, wherein the silica-alumina contains 0.5% to 30% by weight of silica with respect to the anhydrous product.

9. A process according to any one of claims 1 to 8, wherein the group VIII metal content is in the range 0.1 % to 50% by weight.

10. A process according to any one of claims 1 to 9, wherein the group VIII metal is cobalt.

11. A process according to any one of claims 1 to 10, wherein the catalyst contains at least one additional element selected from the group formed by: ruthenium, molybdenum, tantalum, platinum and palladium.

12. A process according to any one of claims 1 to 11, wherein the catalyst further contains 0.1 % to 5% by weight of at least one oxide M₂O₃ of at least one metal M selected from the group formed by lanthanum, praseodymium and neodymium.

13. A process according to any one of claims 1 to 12, wherein the catalyst is used in suspension in a liquid phase, in a three-phase reactor.

14. A process according to claim 13, wherein said catalyst is in the form of a powder with a grain size of less than 800 microns.

15. A process according to any one of claims 1 to 12, wherein the catalyst is used in a fixed bed in the form of particles with an equivalent diameter in the range 2 to 10 mm.

## Patentansprüche

1. Verfahren zur Synthese einer Mischung aus linearen und gesättigten Kohlenwasserstoffen, die mindestens 50 Gew.% C5+-Kohlenwasserstoffe und weniger als 20 % Methan, bezogen auf die Gesamtheit der gebildeten Kohlenwasserstoffe, enthält, aus einer Mischung, die Kohlenmonoxid und Wasserstoff in Gegenwart eines Katalysators umfasst, der mindestens ein Metall der Gruppe VIII, geträgert auf einem Siliciumdioxid-Aluminiumoxid umfasst, hergestellt durch Copräzipitieren und mindestens 6-stündiges Kalzinieren bei einer Temperatur im Bereich zwischen ungefähr 500 °C und ungefähr 1.200 °C, so dass das Siliciumdioxid-Aluminiumoxid eine spezifische Oberfläche von weniger als 260 m2/g aufweist, **dadurch gekennzeichnet, dass** der Siliciumdioxid-Aluminiumoxid-Träger hergestellt wird durch Präzipitieren eines Aluminiumoxid-Hydrogels, wobei dann das gemischte Siliciumdioxid-Aluminiumoxid-Hydrogel durch Zugabe einer Kieselsäurelösung erhalten wird, gründliches Homogenisieren durch starkes Rühren, Scheren, Kolloidmahlen, Trocknen mit Formen, dann Kalzinieren.

2. Verfahren nach Anspruch 1 wobei die Homogenisierung durch Feinvermahlung in einer Kolloidmühle durchgeführt wird.

3. Verfahren nach einem der Ansprüche 1 bis 2, wobei das Siliciumdioxid-Aluminiumoxid bei einer Temperatur im Bereich zwischen 700 °C und 1.200 °C mindestens 10 Stunden lang kalziniert wird.

4. Verfahren nach einem der Ansprüche 1 bis 3, wobei das Siliciumdioxid-Aluminiumoxid zuerst bei einer Temperatur im Bereich zwischen 350 °C und 550°C mindestens 1 Stunde lang, dann bei einer Temperatur im Bereich zwischen ungefähr 500 °C und ungefähr 1.200 °C mindestens 6 Stunden lang kalziniert wird.

5. Verfahren nach einem der Ansprüche 1 bis 4, wobei das Siliciumdioxid-Aluminiumoxid auf Mikrometerskala homogen ist.

6. Verfahren nach einem der Ansprüche 1 bis 5, wobei das Siliciumdioxid-Aluminiumoxid auf Nanometerskala homogen ist.

7. Verfahren nach einem der Ansprüche 1 bis 6, wobei der Gehalt an anionischen und kationischen Verunreinigungen unter 0,1 Gew.-% liegt.

8. Verfahren nach einem der Ansprüche 1 bis 7, wobei das Siliciumdioxid-Aluminiumoxid zwischen 0,5 Gew.-% und 30 Gew.-% Siliciumdioxid bezogen auf das Anhydridprodukt enthält.

9. Verfahren nach einem der Ansprüche 1 bis 8, wobei der Gehalt an einem Metall der Gruppe VIII im Bereich zwischen 0,1 und 50 Gew.-% liegt.

10. Verfahren nach einem der Ansprüche 1 bis 9, wobei das Metall der Gruppe VIII Cobalt ist.

11. Verfahren nach einem der Ansprüche 1 bis 10, wobei der Katalysator mindestens ein zusätzliches Element enthält, ausgewählt aus der Gruppe, bestehend aus: Ruthenium, Molybdän, Tantal, Platin, Palladium.

12. Verfahren nach einem der Ansprüche 1 bis 11, wobei der Katalysator ferner 0,1 bis 5 Gew.-% mindestens eines Oxids M203 mindestens eines Metalls M enthält, ausgewählt aus der Gruppe, bestehend aus Lanthan, Praseodym und Neodym.

13. Verfahren nach einem der Ansprüche 1 bis 12, wobei der Katalysator in Suspension in einer flüssigen Phase in einem Dreiphasenreaktor eingesetzt wird.

14. Verfahren nach Anspruch 13, wobei der Katalysator die Form eines Pulvers hat, das eine Korngröße von weniger als 800 Mikron aufweist.

15. Verfahren nach einem der Ansprüche 1 bis 12, wobei der Katalysator in einem Festbett in Form von Teilchen mit äquivalenten Durchmesser zwischen 2 und 10 mm eingesetzt wird.
